# EUROPÄISCHE PATENTSCHRIFT

(11) **EP 3 738 534 B1**
(45) Veröffentlichungstag und Bekanntmachung des Hinweises auf die Patenterteilung: **27.04.2022**
(21) Anmeldenummer: 20167636.8
(22) Anmeldetag: 02.04.2020
(51) Int. Cl.: A61B 17/80

(54) **EINRICHTUNG ZUM FIXIEREN VON KNOCHEN**
BONE FIXATION DEVICE
DISPOSITIF DE FIXATION DES OS

(30) Priorität: 03.04.2019 DE 102019108777
(43) Veröffentlichungstag der Anmeldung: 18.11.2020
(73) Patentinhaber: Osteobionix SL, 35001 Las Palmas de Gran Canaria Las Palmas (ES)
(72) Erfinder: Monopoli, Donato, 35001 LAS PALMAS DE GRAN CANARIA (ES); Mentado Almeida, Belinda, 35009 LAS PALMAS DE GRAN CANARIA (ES); Afonso Martín, Hernando Oliver, 35415 ARUCAS, LAS PALMAS (ES)
(74) Vertreter: DREISS Patentanwälte PartG mbB

(56) Entgegenhaltungen:
- EP-A1- 2 319 437
- EP-A1- 2 719 348
- US-A1- 2005 043 736
- US-A1- 2008 119 895
- US-A1- 2008 306 550

## Beschreibung

Die Erfindung betrifft eine Einrichtung zum Fixieren von Knochen mit einer Knochenplatte, die wenigstens ein Durchgangsloch aufweist. Im Durchgangsloch ist ein Ausrichtmittel beweglich angeordnet, das ein Innengewinde zum Einschrauben eines an einem Schraubenkopf einer Knochenschraube vorgesehenen Außengewinde aufweist, so dass bei eingeschraubter Knochenschraube das Ausrichtmittel an der Knochenplatte geklemmt festgesetzt ist. Derartige Einrichtungen werden auch als Osteosyntheseeinrichtungen bezeichnet.

Eine derartige Einrichtung aus der EP 2 644 141 B1 bekannt. Das Ausrichtmittel ist hier als Schieber ausgebildet, dass entlang einer parallel zur Knochenplatte verlaufenden Achse in einem Gleitloch beweglich angeordnet ist. Das Ausrichtmittel, und damit die Knochenschraube, kann um die Mittelachse des Gleitlochs verschenkt werden. Ein freies Verschwenken der Knochenschraube um unterschiedliche Schwenkachsen ist nicht möglich.

Aus der DE 43 43 117 A1 ist ein Fixationssystem für Knochen bekannt geworden, welches eine Knochenplatte mit wenigstens einem Durchgangsloch für eine Knochenschraube aufweist. Dabei soll durch geeignete Form des Durchgangsloches und einer kugeligen Form des Schraubenkopfes gewährleistet sein, dass die Schraube nicht nur koaxial in das Durchgangsloch, sondern in einem wählbaren Winkel, und damit um unterschiedliche Schwenkachsen verschwenkbar einschraubbar ist.

Den bekannten Einrichtungen und Systemen haftet der Nachteil an, dass sie nicht einfach herstellbar und in der Anwendung nicht einfach handzuhaben sind.

In der DE 100 15 734 A1 ist ein Befestigungssystem von Implantaten mittels Schraube und Spreizring offenbart, wobei ein Lagerring und der Spreizring korrespondierende Oberflächen aufweisen, sodass die Schraube in verschiedenen Winkelstellungen befestigt werden kann. Aus der US 2008 0119895 A1 ist ein Befestigungssystem für Implantate an Knochen mittels Spreizring und Schraube bekannt, wobei der Spreizring und das Implantat ebenfalls korrespondierende Oberflächen aufweisen. Zudem dient ein Halteelement zur Drehsicherung des Spreizrings.

Der Erfindung liegt die Aufgabe zugrunde, eine Einrichtung zum Fixieren von Knochen der eingangs genannten Art so weiterzubilden, dass sie einerseits einfach herstellbar ist, andererseits aber auch gewährleistet ist, dass die Knochenschraube unter verschiedenen Winkeln bzw. um verschiedene Schwenkachsen schwenkbar einschraubbar ist. Diese Aufgabe wird mit einer Einrichtung mit den Merkmalen des Anspruchs 1 gelöst. Dabei ist vorgesehen, dass das Durchgangsloch eine Kugeloberfläche aufweist, deren Mittelpunkt auf der Lochachse liegt, und dass das Ausrichtmittel als Ausrichtring ausgebildet ist, der eine zur Kugeloberfläche des Durchgangslochs wenigstens abschnittsweise komplementäre äußere Oberfläche aufweist, so dass der Ausrichtring im Durchgangsloch um unterschiedliche Schwenkachsen verschwenkbar gelagert angeordnet ist. Dadurch kann erreicht werden, dass die Ringachse, und damit der Ausrichtring, innerhalb eines Bereichs in einen beliebigen Winkel verstellt werden kann, wodurch die Knochenschraube letztlich unter verschiedenen Winkeln verschraubt werden kann. Um ein Verschwenken des Ausrichtrings in eine beliebige Winkelstellung zu ermöglichen, ist es ausreichend, wenn der Ausrichtring gegenüber der Knochenplatte um zwei Schwenkachsen verschwenkbar gelagert ist. Eine Schwenkachse kann beispielsweise parallel zur Ober- oder Unterseite der Knochenplatte verlaufen; eine zweite Schwenkachse kann dazu senkrecht verlaufen. Die Herstellung einer solchen Einrichtung ist vergleichsweise einfach; das Durchgangsloch mit der Kugeloberfläche ist bereitzustellen und der Ausgleichsring ist in das Durchgangsloch einzusetzen, so dass dieser im Durchgangsloch um wenigstens zwei Schwenkachsen verschwenkbar ist. Beim Einschrauben der Knochenschraube durchgreift diese zunächst den Ausrichtring. Dann, wenn der Schraubenkopf in Kontakt mit dem Innengewinde des Ausrichtrings kommt, wird dieser aufgeweitet oder aufgespreizt, wodurch der Ausrichtring im Durchgangsloch klemmend festgesetzt wird.

Ferner weist der Ausrichtring eine Ausnehmung auf und an der Knochenplatte ist ein Halteelement mit einem in die Ausnehmung eingreifenden Halteabschnitt vorgesehen. Die Ausnehmung ist dabei derart, dass ein Mitverdrehen des Ausrichtrings um seine Ringachse beim Einschrauben der Knochenschraube, also dann, wenn das Außengewinde des Schraubenkopfes mit dem Innengewinde des Ausrichtrings zusammenwirkt, unterbunden wird. Das Vorsehen einer Ausnehmung hat ferner den Vorteil, dass der Ausrichtring in radialer Richtung elastisch nachgiebig ausgebildet werden kann, so dass dieser zum einen in das Durchgangsloch, welches die Kugeloberfläche aufweist, eingesetzt werden kann, und so dass dieser beim Einschrauben des Schraubenkopfes in radialer Richtung zum Festsetzen gegen die Knochenplatte aufgeweitet bzw. gespreizt werden kann. Die Ausnehmung kann dabei insbesondere in einer Radialebene der Ringachse liegen und den Ausrichtring vorzugsweise vollständig durchtrennen, so dass der Ring von der Ausnehmung gespalten oder geschlitzt wird.

Der Ausrichtring ist dabei so angeordnet, dass er im Durchgangsloch wenigstens um die Längsachse des Halteabschnitts des Halteelements verschwenkbar angeordnet ist. Die Anordnung ist dabei vorzugsweise derart, dass die Längsachse des Halteabschnitts durch den Mittelpunkt der Kugeloberfläche des Durchgangslochs verläuft.

In diesem Zusammenhang ist es vorteilhaft, wenn der Ausrichtring im Durchgangsloch zudem um eine senkrecht zur Längsachse des Halteabschnitts verlaufende Querachse verschwenkbar angeordnet ist. Auch diese Querachse verläuft vorzugsweise ebenfalls durch den Mittelpunkt der Kugeloberfläche. Durch die Möglichkeit, den Haltering um die Längsachse des Halteabschnitts und um eine dazu quer verlaufende Querachse zu verschwenken, kann der Ausrichtring letztlich so ausgerichtet werden, dass die Knochenschraube unter einem frei wählbaren Winkel eingeschraubt werden kann.

Eine weitere vorteilhafte Ausführungsform ergibt sich dann, wenn die Ausnehmung des Ausrichtrings von zwei parallelen Führungsflächen begrenzt wird, die vorzugsweise parallel zur Ringachse verlaufen. Ferner ist vorteilhaft, wenn der Halteabschnitt einen wenigstens abschnittsweise runden Querschnitt mit Anlageabschnitten zur Anlage an die Führungsflächen aufweist. Die Anordnung ist vorzugsweise derart, dass ein Verschwenken des Ausrichtrings um die Längsachse des Halteabschnitts und um die dazu senkrecht verlaufende Querachse, die durch den Mittelpunkt der Kugeloberfläche verläuft, ermöglicht wird. Wird folglich der Ausrichtring um die Längsachse des Halteabschnitts verschwenkt, so bleiben die Kontaktpunkte der Führungsflächen die gleichen, wobei diese Kontaktpunkte während einem Verschwenken entlang den Anlageabschnitten des Halteabschnitts erstrecken. Wird der Ausgleichsring um die Querachse verschwenkt, so wandern die Kontaktpunkte an den Führungsflächen bei gleichbleibenden Kontaktpunkten an den Anlageabschnitten. In diesem Zusammenhang ist vorteilhaft, wenn der Abstand der Führungsflächen gleich oder geringfügig kleiner ist als der größte Durchmesser des Halteabschnitts.

Das Halteelement kann beispielsweise einstückig mit der Knochenplatte ausgebildet sein. Allerdings hat sich als vorteilhaft erwiesen, wenn das Halteelement als eigenes Bauteil ausgebildet ist. Dazu kann die Knochenplatte eine Aussparung aufweisen, in welche ein Fixierabschnitt des Halteelements positionsgenau eingesetzt werden kann.

Die Aussparung kann dabei als Zylinderaussparung ausgebildet sein, wobei der Fixierabschnitt dann eine zur Zylinderaussparung komplementäre Kontur aufweist.

Die Knochenplatte als solche kann insbesondere mehrere Durchgangslöcher mit einer Kugeloberfläche aufweisen und mehrere, zugehörige Halteelemente mit entsprechend ausgebildeten Halteabschnitten vorsehen. Dabei hat sich als vorteilhaft erwiesen, wenn die Längsachsen der Halteabschnitte der unterschiedlichen Halteelemente jeweils einen spitzen Winkel α einschließen, der vorzugsweise im Bereich von 5° bis 40° und weiter vorzugsweise im Bereich von 10° bis 20° liegt. Ferner ist vorteilhaft, wenn die Knochenplatte mehrere Durchgangslöcher aufweist, und wenn die Lochachsen insbesondere benachbarter Durchgangslöcher nicht in einer longitudinalen Mittelebene der Knochenplatte liegen, sondern dazu versetzt angeordnet sind. Dadurch können auf die Halteelemente wirkende Kräfte vorteilhaft in die Knochenplatte abgeleitet werden.

Vorteilhafterweise umfasst die erfindungsgemäße Einrichtung wenigstens eine Knochenschraube, die einen Schraubenkopf mit einem Außengewinde aufweist, so dass bei in den Ausrichtring eingeschraubter Knochenschraube der Ausrichtring an der Knochenplatte geklemmt festgesetzt ist. Der Ausrichtring ist dabei vorzugsweise in radialer Richtung elastisch nachgiebig, so dass ein Festsetzen durch Klemmen sicher gewährleistet werden kann.

Dabei ist denkbar, dass das Innengewinde des Ausrichtrings und das Außengewinde des Schraubenkopfs kegelförmig ausgebildet sind. Je tiefer der Schraubenkopf in den Ausrichtring eingeschraubt wird, desto größer sind die auftretenden Klemmkräfte.

Weitere Einzelheiten und vorteilhafte Ausgestaltungen der Erfindung sind der nachfolgenden Beschreibung zu entnehmen, anhand derer ein Ausführungsbeispiel der Erfindung näher beschrieben und erläutert ist.

Es zeigen:
- Figur 1: eine Einrichtung zum Fixieren von Knochen, die eine Knochenplatte mit Knochenschrauben umfasst;
- Figur 2: eine Explosionsansicht eines Ausschnitts der Einrichtung gemäß Figur 1;
- Figur 3: eine Seitenansicht des Ausschnitts gemäß Figur 2;
- Figur 4: die Draufsicht des Ausschnitts gemäß Figur 2;
- Figur 5: einen Querschnitt durch eine an der Knochenplatte festgesetzte Knochenschraube;
- Figur 6: einen Längsschnitt der in der Figur 5 gezeigten Ansicht;
- Figur 7: einen senkrecht zum Schnitt gemäß Figur 5 gezeigten Schnitt; und
- Figur 8: ein Halteelement und einen Ausrichtring als Einzelteil.

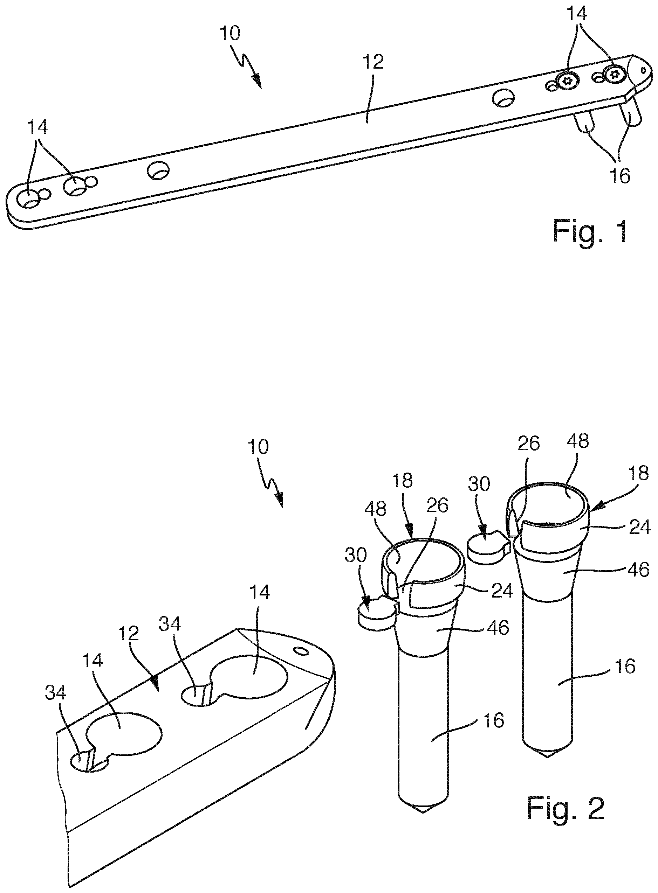

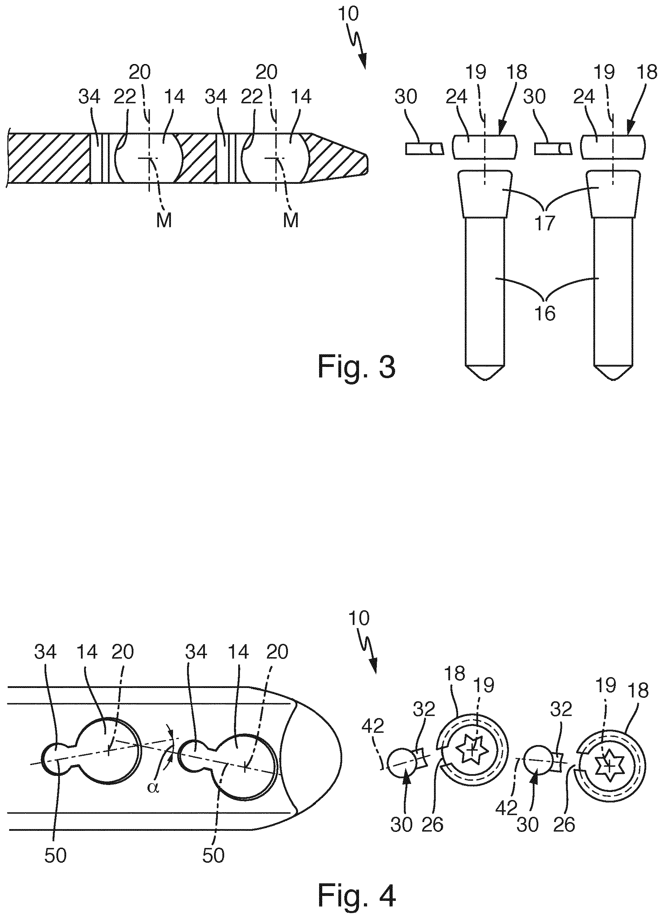

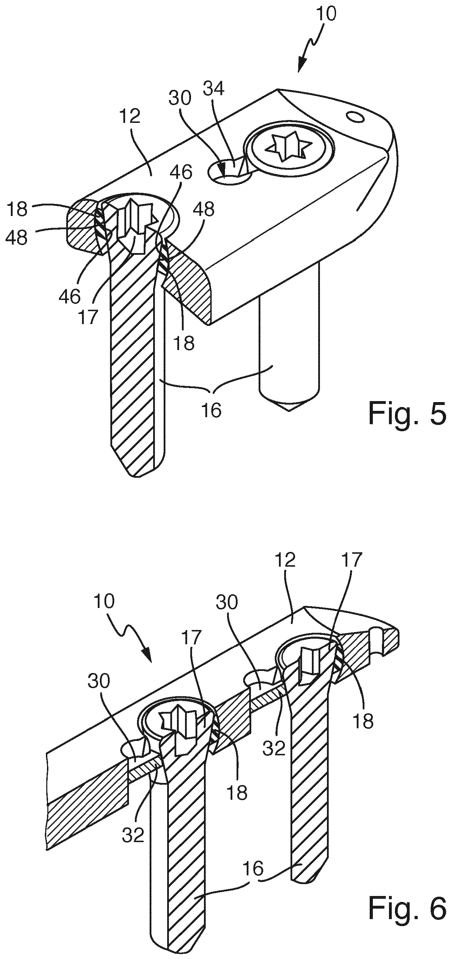

Die in den Figuren gezeigte Einrichtung 10 zum Fixieren von Knochen umfasst eine Knochenplatte 12 mit mehreren Durchgangslöchern 14 zum Einschrauben von Knochenschrauben 16. Die Knochenschrauben 16 weisen jeweils einen Schraubenkopf 17 auf. Wie aus den Explosionszeichnungen gemäß Figur 2, 3 und 4 und aus den Schnitten gemäß Figur 5, 6 und 7 hervorgeht, befindet sich in jedem Durchgangsloch ein Ausrichtring 18. Wie aus dem Schnitt gemäß Figur 3 deutlich wird, weisen die Durchgangslöcher 14 eine Kugeloberfläche 22 auf, deren Mittelpunkt M auf der jeweiligen Lochachse 20 liegt. Die Ausrichtringe 18 weisen eine zu der Kugeloberfläche 22 der Durchgangslöcher 14 komplementäre Kugeloberfläche 24 auf, so dass die Ausrichtringe 18 um den jeweiligen Mittelpunkt der Kugeloberflächen 22 der Durchgangslöcher 14 möglichst spielfrei und dennoch leichtgängig frei verschwenkbar ist.

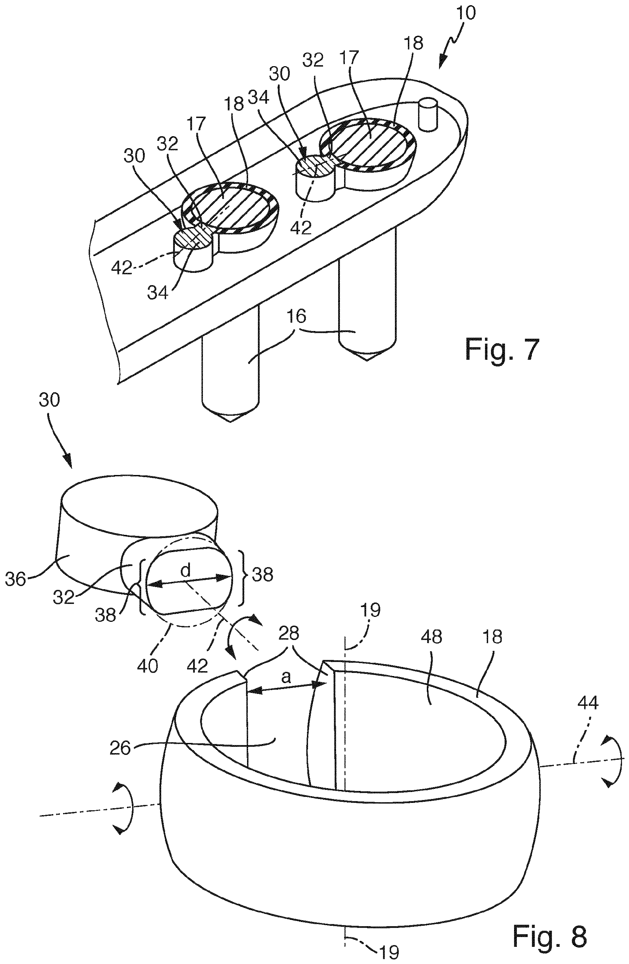

Zur elastischen Nachgiebigkeit der Ausrichtringe 18 sind diese geschlitzt und sehen eine Ausnehmung 26 vor, die insbesondere in Figur 8 deutlich zu erkennen ist. Die Ausnehmungen 26 werden dabei jeweils von zwei parallelen Führungsflächen 28 begrenzt. Die Führungsflächen 28 weisen einen Abstand a zueinander auf. Der Abstand a ist vorzugsweise derart groß, dass die Führungsringe 18 zum Einfügen in die Durchgangslöcher 14 elastisch nachgiebig zusammengedrückt werden können. Im Durchgangsloch 14 verformen sich die Ausrichtringe 18 zurück und liegen vorzugsweise spielfrei an der Kugeloberfläche 22 der Durchgangslöcher 14 an.

Die Einrichtung 10 sieht ferner Halteelemente 30 vor, die jeweils einen Halteabschnitt 32 aufweisen, der im montierten Zustand, wie insbesondere aus Figur 6 und 7 deutlich wird, in die jeweilige Ausnehmung 26 des jeweiligen Ausrichtrings 18 eingreift. Bei der in den Figuren gezeigten Ausführungsform ist das Halteelement 30 als separates Bauteil ausgebildet; denkbar ist auch, dass das Halteelement 30 einstückig mit der Knochenplatte 12 ausgebildet ist.

Zur positionsgenauen und sicheren Anordnung des jeweiligen Halteelements 30 an der Knochenplatte 12 weist die Knochenplatte 12 zylinderförmige Aussparungen 34 auf. Die Halteelemente 30 sehen einen Fixierabschnitt 36 vor, der komplementär zur Kontur der Aussparungen 34 ausgebildet ist, so dass die Fixierabschnitte 36 der Halteelemente 30 in die jeweilige Aussparung 34 eingesetzt und dort fixiert werden kann.

Wie insbesondere aus Figur 8 deutlich wird, weist der jeweilige Halteabschnitt 32 zwei einander gegenüberliegende, um die Längsachse 42 des Halteabschnitts 32 verlaufende und auf einer Kreisbahn 40 liegende Anlageabschnitte 38 auf. Der Abstand d der Anlageabschnitte 38, bzw. der Durchmesser der Kreisbahn 40 ist dabei gleich oder geringfügig kleiner dem Abstand a der Führungsflächen 28. Dadurch wird gewährleistet, dass die Ausrichtringe 18 um die Längsachse 42 des jeweiligen Halteabschnitts 32 geführt verschwenkbar sind. Dadurch, dass der Abstand d der Anlageabschnitte 38 gleich oder geringfügig kleiner dem Abstand a der Führungsflächen 28 ist, wandert beim Verschwenken des Ausrichtrings 18 um die Längsachse 42 des Halteabschnitts 32 der Kontaktpunkt zwischen dem Halteabschnitt 32 und den Führungsflächen 28 auf den Anlageabschnitten 38.

Die Anlageabschnitte 38 dienen ferner zur Führung des Ausrichtrings 18 beim Schwenken um eine quer zur Längsachse 42 verlaufende Querachse 44, die insbesondere in Figur 8 angedeutet ist. Beim Schwenken des Ausrichtrings 18 um die Querachse 44 wandern folglich die Berührpunkte der Anlageabschnitte 38 auf den Führungsflächen 28.

Durch die beschriebene Anordnung kann folglich der jeweilige Ausrichtring 18 zum einen um die Längsachse 42 des jeweiligen Halteabschnitts 32 und zum anderen um eine quer dazu verlaufende Querachse 44 verschwenkt werden. Dadurch kann der Ausrichtring 18, bzw. dessen Ringachse 19, welche letztlich der Schraubenachse entspricht, in einen weitgehend beliebigen Winkel zur Knochenplatte 12 ausgerichtet werden.

Zur Fixierung von Knochen kann in einem ersten Schritt der jeweilige Ausrichtring 18 in richtige Position durch Verschwenken um die jeweilige Längsachse 42 und/oder Drehachse 44 ausgerichtet werden. In einem nächsten Schritt kann die jeweilige Knochenschraube 16 in den jeweiligen Ausrichtring 18 eingeführt und mit dem jeweiligen Knochen verschraubt werden. Das Einschrauben erfolgt so lange, bis der jeweilige Schraubenkopf 17, bzw. ein am Schraubenkopf 17 angeordnetes kegelförmiges Außengewinde 46, mit einem am jeweiligen Ausrichtring 18 vorgesehenen, dazu komplementär ausgebildeten Innengewinde 48 in Eingriff kommt. Aufgrund der kegelförmigen Ausbildung des Außengewindes 46 und des Innengewindes 48 wird beim Einschrauben der jeweiligen Knochenschraube 16 der jeweilige Ausrichtring 18 in radialer Richtung expandiert, so dass dieser in der vorgegebenen Ausrichtung in dem jeweiligen Durchgangsloch 14 geklemmt festgesetzt wird. Das jeweilige Außengewinde 46 sowie das zugehörige Innengewinde 48 sind in den Figuren nicht näher gezeigt.

Wie insbesondere aus der Draufsicht nach Figur 4 deutlich wird, verlaufen die Längsachsen 42 der Halteabschnitte 32 bzw. die Längsachsen 50 der Aussparungen 34 nicht entlang einer Linie oder parallel zueinander, sondern schließen einen spitzen Winkel α von ca. 25° ein. Ferner liegen die Lochachsen 20 der Durchgangslöcher 14 nicht in der longitudinalen Mittelebene der Knochenplatte 12, sondern sind versetzt dazu angeordnet. Dadurch können auf die Halteelemente 30 wirkende Kräfte vorteilhaft in die Knochenplatte 12 abgeleitet werden.

Die Platte 12 kann insbesondere im mittleren Bereich Befestigungsabschnitte oder Löcher zur Befestigung von Distanzstücken (Spacer) aufweisen. Die Distanzstücke sind dabei vorzugsweise aus einem porösen Material und sind zur Knochenrekonstruktion vorgesehen.

Die beschriebene Einrichtung 10 hat folglich den Vorteil, dass Knochenschrauben 16 unterschiedlich ausgerichtet auf einfache Art und Weise mit dem zu fixierenden Knochen verbunden werden können, wobei die Knochenschrauben 16 in ihrer jeweiligen Ausrichtung auf einfache Art und Weise an der Knochenplatte 12 festgesetzt werden.

## Patentansprüche

1. Einrichtung (10) zum Fixieren von Knochen,
mit einer Knochenplatte (12), die wenigstens ein sich um eine Lochachse (20) erstreckendes Durchgangsloch (14) aufweist, und
mit einem im Durchgangsloch (14) beweglich angeordneten Ausrichtmittel (18), das ein Innengewinde (48) zum Einschrauben eines an einem Schraubenkopf (17) einer Knochenschraube (16) vorgesehenen Außengewinde (46) aufweist, so dass bei eingeschraubter Knochenschraube (16) das Ausrichtmittel (18) an der Knochenplatte (12) geklemmt festgesetzt ist,
wobei das Durchgangsloch (14) wenigstens abschnittsweise eine Kugeloberfläche (22) aufweist oder bildet, deren Mittelpunkt (M) auf der Lochachse (20) liegt, wobei das Ausrichtmittel als Ausrichtring (18) ausgebildet ist, der eine zur Kugeloberfläche (22) des Durchgangslochs (14) wenigstens abschnittsweise komplementäre äußere Oberfläche (24) aufweist, so dass der Ausrichtring (18) im Durchgangsloch (14) um unterschiedliche Schwenkachsen (42, 44) verschwenkbar gelagert angeordnet ist,
wobei der Ausrichtring (18) eine Ausnehmung (26) aufweist und
wobei an der Knochenplatte (12) ein Haltelement (30) mit einem in die Ausnehmung (26) eingreifenden Halteabschnitt (32) vorgesehen ist,
so dass ein Mitverdrehen des Ausrichtrings (18) um die Ringachse (19) beim Einschrauben der Knochenschraube (16) unterbunden wird, **dadurch gekennzeichnet,**
**dass** die Anordnung derart ist, dass bei in die Ausnehmung (26) eingreifenden Halteelement (30) der Ausrichtring (18) im Durchgangsloch (14) wenigstens um die Längsachse (42) des Halteabschnitts (32) verschwenkbar ist.

2. Einrichtung (10) nach Anspruch 1, **dadurch gekennzeichnet, dass** der Ausrichtring (18) im Durchgangsloch (14) um eine senkrecht zur Längsachse (42) des Halteabschnitts (32) verlaufende Querachse (44) verschwenkbar angeordnet ist.

3. Einrichtung (10) nach Anspruch 1 oder 2, **dadurch gekennzeichnet, dass** die Ausnehmung (26) des Ausrichtrings (18) von zwei parallelen Führungsflächen (28) begrenzt wird und dass der Halteabschnitt (32) einen wenigstens abschnittsweise runden Querschnitt mit Anlageabschnitten (38) zur Anlage an die Führungsflächen (28) aufweist, so dass ein Verschwenken um die Längsachse (42) des Halteabschnitts (32) und um die dazu senkrecht verlaufende Querachse (44) ermöglicht wird.

4. Einrichtung (10) nach Anspruch 1, 2 oder 3, **dadurch gekennzeichnet, dass** die Knochenplatte (12) eine Aussparung (34) für das Halteelement (30) aufweist und dass das Halteelement (30) einen Fixierabschnitt (36) zum Einsetzen in die Aussparung (34) aufweist.

5. Einrichtung (10) nach Anspruch 4, **dadurch gekennzeichnet, dass** die Aussparung (34) als Zylinderaussparung ausgebildet ist, und dass der Fixierabschnitt (36) des Halteelements (30) eine zur Zylinderaussparung komplementäre Kontur aufweist.

6. Einrichtung (10) nach einem der Ansprüche 1 bis 4, **dadurch gekennzeichnet, dass** die Knochenplatte (12) mehrere Durchgangslöcher (14) und zugehörige Halteelemente (30) mit Halteabschnitten (32) aufweist, wobei die Längsachsen (42) benachbarter Halteabschnitte (32) jeweils einen spitzen Winkel α einschließen, der vorzugsweise im Bereich von 5° bis 40° und weiter vorzugsweise im Bereich von 10° bis 20° liegt.

7. Einrichtung (10) nach einem der Ansprüche 1 bis 4, **dadurch gekennzeichnet, dass** die Knochenplatte (12) mehrere Durchgangslöcher (14) aufweist, wobei die Lochachsen (20) der Durchgangslöcher (14) nicht in einer longitudinalen Mittelebene der Knochenplatte (12) liegen.

8. Einrichtung (10) nach einem der vorhergehenden Ansprüche, **dadurch gekennzeichnet, dass** die Einrichtung (10) wenigstens eine Knochenschraube (16) aufweist, die einen Schraubenkopf (17) mit einem Außengewinde (46) aufweist, so dass bei in den Ausrichtring (18) eingeschraubter Knochenschraube (16) der Ausrichtring (18) an der Knochenplatte (12) geklemmt festgesetzt ist.

9. Einrichtung (10) nach Anspruch 8, **dadurch gekennzeichnet, dass** das Innengewindes (48) des Ausrichtrings (18) und das Außengewinde (46) des Schraubenkopfs (17) kegelförmig ausgebildet sind.

## Claims

1. Device (10) for fixing bones,
with a bone plate (12) which has at least one through hole (14) extending about a hole axis (20), and
with an alignment means (18) which is movably arranged in the through hole (14) and which has an internal thread (48) for screwing in an external thread (46) provided on a screw head (17) of a bone screw (16), so that when the bone screw (16) is screwed in, the alignment means (18) is fixed clamped to the bone plate (12),
wherein the through hole (14) having or forming, at least in sections, a spherical surface (22) whose center (M) is located on the hole axis (20),
wherein the alignment means is formed as an alignment ring (18) having an outer surface (24) complementary at least in sections to the spherical surface (22) of the through hole (14), so that the alignment ring (18) is arranged in a pivoted manner about different pivot axes (42, 44) in the through hole (14), wherein the alignment ring (18) has a recess (26) and wherein a retaining element (30) with a retaining section (32) engaging in the recess (26) is provided on the bone plate (12) so that co-rotation of the alignment ring (18) about the ring axis (19) is prevented when the bone screw (16) is screwed in,
**characterized in that**
the configuration is such that, when the retaining element (30) engages in the recess (26), the alignment ring (18) in the through hole (14) is pivotable at least about the longitudinal axis (42) of the retaining section (32).

2. Device (10) according to claim 1, **characterized in that** the alignment ring (18) is arranged in the through hole (14) pivotably about a transverse axis (44) extending perpendicularly to the longitudinal axis (42) of the retaining section (32).

3. Device (10) according to claim 1 or 2, **characterized in that** the recess (26) of the alignment ring (18) is delimited by two parallel guide surfaces (28), and **in that** the retaining section (32) has an at least in sections round cross-section with abutment sections (38) for abutment against the guide surfaces (28), so that pivoting about the longitudinal axis (42) of the retaining section (32) and about the transverse axis (44) extending perpendicular thereto is facilitated.

4. Device (10) according to claim 1, 2 or 3, **characterized in that** the bone plate (12) has a notch (34) for the retaining element (30) and that the retaining element (30) has a fixing section (36) for insertion into the notch (34).

5. Device (10) according to claim 4, **characterized in that** the notch (34) is formed as a cylinder notch, and **in that** the fixing section (36) of the retaining element (30) has a contour complementary to the cylinder notch.

6. Device (10) according to any one of claims 1 to 4, **characterized in that** the bone plate (12) has a plurality of through holes (14) and corresponding retaining elements (30) with retaining sections (32), wherein the longitudinal axes (42) of adjacent retaining sections (32) each enclose an acute angle a, which is preferably in the range of 5° to 40° and further preferably in the range of 10° to 20°.

7. Device (10) according to any one of claims 1 to 4, **characterized in that** the bone plate (12) has a plurality of through holes (14), wherein the hole axes (20) of the through holes (14) are not located in a longitudinal center plane of the bone plate (12).

8. Device (10) according to any one of the preceding claims, **characterized in that** the device (10) has at least one bone screw (16) having a screw head (17) with an external thread (46), so that when the bone screw (16) is screwed into the alignment ring (18), the alignment ring (18) is fixed clamped to the bone plate (12).

9. Device (10) according to claim 8, **characterized in that** the internal thread (48) of the alignment ring (18) and the external thread (46) of the screw head (17) are conical.

## Revendications

1. Dispositif (10) pour fixer des os,
avec une plaque orthopédique (12), qui présente au moins un trou débouchant (14) s'étendant autour d'un axe de trou (20), et
avec un moyen orientable (18) disposé de manière mobile dans le trou débouchant (14), qui présente un filetage femelle (48) pour le vissage d'un filetage mâle (46) prévu sur une tête de vis (17) d'une vis à os (16), de sorte que lorsque la vis à os (16) est vissée, le moyen orientable (18) est immobilisé de manière coincée sur la plaque orthopédique (12),
dans lequel le trou débouchant (14) présente ou forme au moins en partie une surface sphérique (22), dont le centre (M) se situe sur l'axe de trou (20),
dans lequel le moyen orientable est réalisé sous la forme d'une bague orientable (18), qui présente une surface extérieure (24) complémentaire au moins en partie de la surface sphérique (22) du trou débouchant (14), de sorte que la bague orientable (18) est disposée dans le trou débouchant (14) en étant montée de manière à pouvoir pivoter autour d'axes de pivotement (42, 44) différents,
dans lequel la bague orientable (18) présente un creux (26) et
dans lequel un élément de retenue (30) avec une partie de retenue (32) s'insérant dans le creux (26) est prévu sur la plaque orthopédique (12),
de sorte qu'une rotation conjointe de la bague orientable (18) autour de l'axe de bague (19) lors du vissage de la vis à os (16) est empêchée, **caractérisé en ce que** la disposition est telle que lorsque l'élément de retenue (30) est inséré dans le creux (26), la bague orientable (18) peut pivoter dans le trou débouchant (14) au moins autour de l'axe longitudinal (42) de la partie de retenue (32).

2. Dispositif (10) selon la revendication 1, **caractérisé en ce que** la bague orientable (18) est disposée dans le trou débouchant (14) de manière à pouvoir pivoter autour d'un axe transversal (44) s'étendant perpendiculairement à l'axe longitudinal (42) de la partie de retenue (32).

3. Dispositif (10) selon la revendication 1 ou 2, **caractérisé en ce que** le creux (26) de la bague orientable (18) est délimité par deux surfaces de guidage (28) parallèles et que la partie de retenue (32) présente une section transversale au moins en partie ronde avec des parties d'appui (38) destinées à venir en appui sur les surfaces de guidage (28), de sorte qu'un pivotement autour de l'axe longitudinal (42) de la partie de retenue (32) et autour de l'axe transversal (44) s'étendant perpendiculairement à celui-ci est permis.

4. Dispositif (10) selon la revendication 1, 2 ou 3, **caractérisé en ce que** la plaque orthopédique (12) présente un évidement (34) pour l'élément de retenue (30) et que l'élément de retenue (30) présente une partie de fixation (36) à insérer dans l'évidement (34).

5. Dispositif (10) selon la revendication 4, **caractérisé en ce que** l'évidement (34) est réalisé sous la forme d'un évidement cylindrique, et que la partie de fixation (36) de l'élément de retenue (30) présente un contour complémentaire de l'évidement cylindrique.

6. Dispositif (10) selon l'une quelconque des revendications 1 à 4, **caractérisé en ce que** la plaque orthopédique (12) présente plusieurs trous débouchants (14) et éléments de retenue (30) associés avec des parties de retenue (32), dans lequel les axes longitudinaux (42) de parties de retenue (32) voisines forment respectivement un angle aigu a, qui se situe de préférence dans la plage de 5° à 40° et plus préférablement dans la plage de 10° à 20°.

7. Dispositif (10) selon l'une quelconque des revendications 1 à 4, **caractérisé en ce que** la plaque orthopédique (12) présente plusieurs trous débouchants (14), dans lequel les axes de trou (20) des trous débouchants (14) ne se situent pas dans un plan médian longitudinal de la plaque orthopédique (12).

8. Dispositif (10) selon l'une quelconque des revendications précédentes, **caractérisé en ce que** le dispositif (10) présente au moins une vis à os (16), qui présente une tête de vis (17) avec un filetage mâle (46), de sorte que lorsque la vis à os (16) est vissée dans la bague orientable (18), la bague orientable (18) est immobilisée de manière coincée sur la plaque orthopédique (12).

9. Dispositif (10) selon la revendication 8, **caractérisé en ce que** le filetage femelle (48) de la bague orientable (18) et le filetage mâle (46) de la tête de vis (17) sont réalisés de manière conique.
